(19) Europäisches Patentamt
European Patent Office
Office européen des brevets

(11) Veröffentlichungsnummer: **0 018 504**
**A1**

(12) # EUROPÄISCHE PATENTANMELDUNG

(21) Anmeldenummer: 80101702.1

(22) Anmeldetag: 31.03.80

(51) Int. Cl.[3]: **A 01 N 35/02**
C 11 D 3/48
//C07C45/50, C07C47/115, C07C47/225, C07C47/33, C07C47/347, C07C47/42, C07C47/445, C07C47/45

(30) Priorität: 07.04.79 DE 2914090

(43) Veröffentlichungstag der Anmeldung: 12.11.80 Patentblatt 80/23

(84) Benannte Vertragsstaaten: AT BE CH DE FR GB IT NL SE

(71) Anmelder: **Henkel Kommanditgesellschaft auf Aktien**
**-Patentabteilung- Postfach 1100 Henkelstrasse 67**
**D-4000 Düsseldorf 1(DE)**

(72) Erfinder: **Hagen, Jens, Dr.**
**Dieselstrasse 5**
**D-6834 Ketsch(DE)**

(72) Erfinder: **Lehmann, Rudolf, Dr.**
**Fröbelstrasse 4a**
**D-4040 Neuss(DE)**

(72) Erfinder: **Bansemir, Klaus, Dr.**
**Ursulaweg 51**
**D-4018 Langenfeld(DE)**

(54) **Verwendung von Aldehyden als antimikrobielle Wirkstoffe.**

(57) Die wirksamen Aldehyde werden durch Hydroformylierung alicyclischer Kohlenwasserstoffe ($C_{6-20}$) mit mindestens einer Doppelbindung bei 70-160° C unter einem Druck von 100-400 bar und in Gegenwart eines Katalysatorgemisches aus a) tert. Phosphinen, z.B. Trialkylphosphinen ($C_{1-20}$) und Triphenylphosphin und b) diese Phosphine enthaltenden Rhodiumcarbonylkomplexen, z.B. Rhcl(CO) $[P(C_6H_5)_3]_2$ hergestellt.
Bevorzugte Ausgangsverbindungen sind Limonen, Camphen, ß-Pinen, $\eta$-Terpinen, Bicyclo- [2,2,1]-hepta-2,5-dien, Vinylnorbornen und Cyclooctan.

In den antimikrobiellen Mitteln liegen die Aldehydwirkstoffe in Mengen von 0,1-25 (0,5-20) Gew.% vor, in Konservierungsmitteln in Mengen von 0,1-2 Gew.-%.

Die Reaktionsprodukte sind Gemische aus Stereoisomeren der Aldehydverbindungen. Diese Gemische sind antimikrobiell wirksam und besitzen einen angenehmen Geruch, weshalb sie gut mit unangenehm riechenden Wirkstoffen eingesetzt werden können.

EP 0 018 504 A1

4000 Düsseldorf, den
Henkelstraße 67
dr.gla-sü

00 8504
HENKEL KGaA
ZR-FE/Patente

# Patentanmeldung

## D 5933

**"Verwendung von Aldehyden als antimikrobielle Wirkstoffe"**

Die Erfindung betrifft die Verwendung von Aldehyden, die aus einfach oder mehrfach ungesättigten alicyclischen Kohlenwasserstoffen durch Hydroformylierung hergestellt werden, als antimikrobielle Wirkstoffe.

Es wurde gefunden, daß Aldehyde, die aus alicyclischen Kohlenwasserstoffen mit 6 bis 20 Kohlenstoffatomen und mindestens einer Doppelbindung durch Hydroformylierung in Gegenwart von Rhodiumkatalysatoren hergestellt werden, sich mit Vorteil als antimikrobielle Wirkstoffe verwenden lassen.

Die Herstellung der erfindungsgemäß zu verwendenden Aldehyde erfolgt zweckmäßigerweise durch Hydroformylierung bei 70 bis 160° C unter einem Druck von 100 bis 400 bar und in Gegenwart eines Katalysatorgemisches aus tertiären Phosphinen und diese tertiären Phosphine enthaltenden Rhodiumcarbonylkomplexen.

/2

Als tertiäre Phosphine eignen sich Trialkylphosphine, deren Alkylreste 1 bis 20 Kohlenstoffatome aufweisen, sowie Triphenylphosphine, deren Phenylreste durch Alkyl- oder Alkoxygruppen mit 1 bis 4 Kohlenstoffatomen substituiert sein können, insbesondere jedoch Triphenylphosphin. In den Katalysatormischungen liegt die Molzahl von insgesamt vorhandenem Phosphin pro Grammatom Rhodium im Bereich von 20 bis 200.

Die genaue Zusammensetzung der katalytisch wirksamen Rhodiumcarbonylkomplexe ist nicht bekannt. Man kann jedoch davon ausgehen, daß es sich um Rhodiumkomplexe handelt, in denen ein oder mehrere Carbonylliganden durch Phosphinliganden ersetzt sind. Die tatsächlich wirksame Komplexverbindung wird in jedem Fall in situ unter den Bedingungen der Hydroformylierung gebildet. Die hierzu erforderliche Menge Rhodium kann deshalb dem Reaktionsgemisch in Form von Rhodiumchlorid, Rhodiumoxid, Rhodiumsalzen von Fettsäuren, Rhodiumchelaten, Rhodiumcarbonyl oder dimerem Rhodiumcarbonylchlorid zugeführt werden. Vorzugsweise setzt man Rhodiumkomplexe ein, die das im Katalysatorgemisch vorhandene Phosphin bereits als Ligand enthalten, beispielsweise die Verbindung $RhCl(CO)[P(C_6H_5)_3]_2$.

Die Rhodiumverbindungen setzt man mit Vorteil in solchen Mengen ein, daß, bezogen auf den zu hydroformylierenden alicyclischen Kohlenwasserstoff, 5 bis 5 000 ppm, vorzugsweise 15 bis 400 ppm, berechnet als Metall, vorhanden sind.

/3

Das hier beschriebene Verfahren hat den Vorteil, daß dabei die unter den Bedingungen der Hydroformylierungsreaktion normalerweise eintretende Isomerisierung der Doppelbindungen weitgehend unterdrückt wird.

Als Ausgangsamterial für die Herstellung der erfindungsgemäß zu verwendenden Aldehyde dienen alicyclische Kohlenwasserstoffe mit 6 bis 20 Kohlenstoffatomen und einer oder mehreren Doppelbindungen. Diese Kohlenwasserstoffe können monocyclische, bicyclische oder polycyclische Konfiguration besitzen; ihre Doppelbindungen können endocyclisch, semicyclisch oder exocyclisch angeordnet sein. Beispiele für solche alicyclischen Verbindungen sind monocyclische und bicyclische Terpene mit mindestens einer Doppelbindung wie die isomeren Menthene, α-Terpinen, β-Terpinen, γ-Terpinen, α-Phellandren, β-Phellandren, Dipenten, Limonen, Pseudolimonen, Terpinolen, α-Pinen, β-Pinen und Camphen; Diels-Alder-Addukte wie Bicyclo[2,2,1]hepta-2,5-dien und dessen Dimeres, sowie Vinylnorbornen; ferner Cycloolefine wie Cyclohexen, Methylcyclohexen, Cycloocten und Cyclooctadien.

Die Hydroformylierung der ungesättigten alicyclischen Kohlenwasserstoffe kann in Abwesenheit von Lösungsmitteln durchgeführt werden. Es hat sich jedoch in vielen Fällen als zweckmäßig erwiesen, Lösungsmittel zu verwenden, wobei u.a. gesättigte Kohlenwasserstoffe wie Pentan, Hexan, Heptan, Cyclohexan, Benzol, Toluol und Xylol, Ether wie Tetrahydrofuran und Dioxan, Alkohole wie Methanol, Ethanol und Isopropanol oder Diole wie Ethylenglykol und Propylenglykol in Frage kommen. Bevorzugt wird die Hydroformylierung in gesättigten Kohlenwasserstoffen oder Ethern durchgeführt.

/4

Die Aufarbeitung des Reaktionsgemisches erfolgt auf dem Wege der Destillation, die zweckmäßigerweise in einer Inertgasatmosphäre, z. B. in einer Stickstoffatmosphäre durchgeführt wird.

Die bei der beschriebenen Hydroformylierung entstehenden Reaktionsprodukte stellen in der Regel Gemische aus den möglichen Stereoisomeren der gewünschten Aldehydverbindungen dar. Diese Gemische sind antimikrobiell wirksam und zeichnen sich druch einen angenehmen Geruch aus. Die durch Hydroformylierung von alicyclischen ungesättigten Kohlenwasserstoffen gewonnenen Aldehyde werden deshalb bevorzugt in Kombination mit unangenehm riechenden antimikrobiellen Wirkstoffen eingesetzt, um deren störenden Geruch zu maskieren.

Bei der Verwendung in antimikrobiellen Mitteln können die Aldehyde in flüssige, pastenförmige oder feste Zubereitungen eingearbeitet werden, z.B. in wäßrige Lösungen, Suspensionen, Emulsionen und Lösungen in organischen Lösungsmitteln. Derartige antimikrobielle Mittel können auf den verschiedensten Gebieten zum Einsatz gelangen, beispielsweise als Reinigungs-, Desinfektions- und Konservierungsmittel für Textilien, Fußböden, Krankenhauseinrichtungen und -instrumente, gewerbliche Betriebe, wie Molkereien, Brauereien und Wäschereien. In antimikrobiellen Mitteln werden die erfindungsgemäß zu verwendenden Substanzen in Mengen von 0,1 bis 25 Gew.-%, vorzugsweise 0,5 bis 20 Gew.-%, bezogen auf das gesamte antimikrobielle Mittel, eingesetzt.

/5

Außerdem können die Aldehyde zur Konservierung von technischen Produkten dienen, die dem Befall durch Bakterien und Pilzen oder sonstiger mikrobieller Zerstörung ausgesetzt sind, beispielsweise Stärkekleistern, Leimen, Dispersionsfarben sowie Schneid- und Bohrölen. Für die Konservierung ist im allgemeinen ein Zusatz von 0,1 bis 2 Gew.-%, bezogen auf das zu konservierende Material, ausreichend.

Die nachfolgenden Beispiele sollen den Gegenstand der Erfindung näher erläutern, ohne ihn hierauf zu beschränken.

/6

## B e i s p i e l e

### Beispiel 1
### Hydroformylierung von Limonen

In einem 5 l-Hubrührautoklaven aus rostfreiem Stahl wurden 1 360 g (10 Mol) Limonen, 15,2 g (58 mMol) Triphenylphosphin und 0,4 g (0,58 mMol) $RhCl(CO)[P(C_6H_5)_3]_2$ miteinander vermischt. Der Autoklav wurde mit Synthesegas gespült. Anschließend wurde ein aus gleichen Volumina Wasserstoff und Kohlenmonoxid bestehendes Gemisch bis zu einem Druck von 200 bar aufgepreßt. Der Autoklaveninhalt wurde unter Rühren auf $125^o$ C aufgeheizt, anschließend unter weiterem Rühren 3 Stunden lang auf dieser Temperatur gehalten, wobei der Druck auf maximal 270 bar anstieg, dann auf Raumtemperatur abgekühlt. Das erhaltene Rohprodukt (1 660 g) wurde im Vakuum unter Stickstoffatmosphäre destilliert. Nach Abtrennen des nicht umgesetzten Limonens gingen bei 111 - $112^o$ C/16 mbar 1 381 g 3-(4-Methyl-3-cyclohexenyl)butyraldehyd (Substanz A; 83 % d. Th.) über.

Die Jodzahl des Produktes betrug 155 (theor. 153). Nach der gaschromatographischen Untersuchung wies das Produkt eine Reinheit von 98 % auf. Das Produkt zeigte folgendes IR-Spektrum (Film):

3 005 $cm^{-1}$; 1 680 $cm^{-1}$ ($>C=C\langle_{H}$);
2 710 $cm^{-1}$; 1 726 $cm^{-1}$ (CHO).



BAD ORIGINAL

## Beispiel 2

## Hydroformylierung von Camphen

408 g (3 Mol) Camphen, 7,6 g (29 mMol) Triphenylphosphin und 0,2 g (0,29 mMol) $RhCl(CO)[P(C_6H_5)_3]_2$ wurden in einem 5 l-Hubrührautoklaven miteinander vermischt. Der Autoklav wurde mit Synthesegas gespült. Anschließend wurde ein aus gleichen Volumina Wasserstoff und Kohlenmonoxid bestehendes Gemisch bis zu einem Druck von 200 bar aufgepreßt. Der Autoklaveninhalt wurde unter Rühren auf 120° C aufgeheizt und unter weiterem Rühren 4 Stunden lang auf 120 bis 130° C gehalten. Nach dem Abkühlen wurde das erhaltene Rohprodukt destillativ aufgearbeitet. Bei 105 - 106° C/20 mbar gingen 383 g 3,3-Dimethyl-2-norbornanacetaldehyd (Substanz B; 77 % d. Th.) über.

Nach der gaschromatographischen Untersuchung bestand das Produkt zu 98 % aus einem Gemisch des endo- und des exo-Stereoisomeren.

Das Produkt zeigte folgendes IR-Spektrum (Film):

2 710 $cm^{-1}$; 1 728 $cm^{-1}$ (CHO); 1 385 $cm^{-1}$; 1 365 $cm^{-1}$ (gem. di-Methyl).

/8

BAD ORIGINAL

Beispiel 3

Hydroformylierung von β-Pinen

Ein 5 l-Hubrührautoklav wurde mit 272 g (2 Mol) β-Pinen, 7,6 g (29 mMol) Triphenylphosphin, 0,2 g (0,29 mMol) $RhCl(CO)[P(C_6H_5)_3]_2$ und 750 ml Tetrahydrofuran beschickt und mit Synthesegas gespült. Anschließend wurde ein aus gleichen Volumina Wasserstoff und Kohlenmonoxid bestehendes Gasgemisch bis zu einem Druck von 200 bar aufgepreßt. Die Reaktionsmischung wurde unter Rühren auf 130° C aufgeheizt und 2 Stunden lang auf dieser Temperatur gehalten. Nach dem Abkühlen wurde das erhaltene Rohprodukt destillativ aufgearbeitet. Bei 105 - 107° C/20 mbar gingen 222 g 10-Formylpinan (Substanz C; 67 % d. Th.) über.

Nach der gaschromatographischen Untersuchung bestand das Produkt zu 97 % aus einem Gemisch aus dem axialen und dem äquatorialen Stereoisomeren.

Das Produkt zeigte folgendes IR-Spektrum (Film):

2 710 $cm^{-1}$; 1 725 $cm^{-1}$ (CHO); 1 368 $cm^{-1}$; 1 381 $cm^{-1}$ (gem. di-Methyl).

/9

Beispiel 4

Hydroformylierung von α-Terpinen

In einem 5 l-Hubrührautoklaven wurden 272 g (2 Mol) α-Terpinen, 3,8 g (14,5 mMol) $P(C_6H_5)_3$, 0,2 g (0,29 mMol) $RhCl(CO)[P(C_6H_5)_3]_2$ und 750 ml Tetrahydrofuran miteinander vermischt. Der Autoklav wurde mit Synthesegas gespült. Anschließend wurde ein aus gleichen Volumina Wasserstoff und Kohlenmonoxid bestehendes Gemisch bis zu einem Druck von 200 bar aufgepreßt. Der Autoklaveninhalt wurde unter Rühren auf 130° C aufgeheizt, anschließend 5 Stunden lang auf 130 - 140° C gehalten, dann auf Raumtemperatur abgekühlt. Aus dem Reaktionsgemisch wurde Tetrahydrofuran im Wasserstrahlvakuum abdestilliert. Bei der Destillation des Rückstandes im Ölpumpenvakuum gingen bei 98 - 100° C / 20 mbar 225 g Produkt (Substanz D; 68 % d. Th.) über.

Die gaschromatische Untersuchung zeigte, daß das Produkt ein Gemisch aus 2-Formyl-Δ3-menthen und 3-Formyl-Δ1-menthen darstellt.

Das Produkt zeigte folgendes IR-Spektrum (Film):

3 005 $cm^{-1}$; 1 682 $cm^{-1}$ ($>C=C<_H$); 2 700 $cm^{-1}$; 1 725 $cm^{-1}$ (CHO); 1 380 $cm^{-1}$; 1 360 $cm^{-1}$ (Isopropyl); 845 $cm^{-1}$ (C=C trisubstituiert).



BAD ORIGINAL

Beispiel 5

Hydroformylierung von Bicycloheptadien-2,5

In einem 5 l-Hubrührautoklaven aus rostfreiem Stahl wurden 460 g (5 Mol) Bicyclo[2,2,1]hepta-2,5-dien, 0,4 g (0,58 mMol) $RhCl(CO)[P(C_6H_5)_3]_2$, 7,6 g (29 mMol) Triphenylphosphin und 500 ml Tetrahydrofuran miteinander vermischt. Der Autoklav wurde mit Synthesegas gespült und anschließend wurde ein aus gleichen Volumina Wasserstoff und Kohlenmonoxid bestehendes Gemisch bis zu einem Druck von 200 bar aufgepreßt. Der Autoklaveninhalt wurde unter Rühren auf 120°C aufgeheizt, anschließend unter wieterem Rühren 5 Stunden lang auf 120 - 130° C gehalten, wobei der Druck maximal auf 270 bar anstieg und dann auf Raumtemperatur abgekühlt. Nach gaschromatographischer Analyse bestand das Rohprodukt aus 3 % nicht umgesetztem Olefin, 8 % Monoaldehyden und 89 % Dialdehyden.

Lösungsmittel und nicht umgesetztes Olefin wurden im Wasserstrahlvakuum abgezogen, das erhaltene Rohprodukt (750 g) wurde anschließend im Vakuum unter Stickstoffatmosphäre destilliert. Nach Abtrennen der Monoaldehyde gingen bei 127 - 130° C/18 mbar 546 g Dialdehyde (Substanz E; 72 % d. Th.) über.

Die CO-Zahl des Produktes betrug 354 (theor. 368). Nach der gaschromatographischen Untersuchung wies das Produkt eine Reinheit von 98 % auf.

/11

Beispiel 6

Hydroformylierung von dimerem Bicycloheptadien

In einem 5 l-Hubrührautoklaven wurden 150 g (0,82 Mol) dimeres Bicycloheptadien, 0,1 g (0,14 mMol) $RhCl(CO)[P(C_6H_5)_3]_2$, 3,8 g (14,5 mMol) Triphenylphosphin und 750 ml Tetrahydrofuran miteinander vermischt. Der Autoklav wurde mit Synthesegas gespült. Danach wurde ein aus gleichen Volumina Wasserstoff und Kohlenmonoxid bestehendes Gemisch bis zu einem Druck von 200 bar aufgepreßt. Unter Rühren wurde der Autoklaveninhalt auf 130° C aufgeheizt, unter weiterem Rühren 1 1/2 Stunden lang auf 120 - 140° C gehalten und schließlich auf Raumtemperatur abgekühlt. Das Lösungsmittel wurde im Wasserstrahlvakuum abdestilliert. Das zurückbleibende Rohprodukt bestand nach gaschromatographischer Analyse aus 4 % Olefin und Paraffin, 86 % Monoaldehyden und 10 % Dialdehyden. Bei der Vakuumdestillation unter Stickstoffatmosphäre gingen bei 130 - 140° C/0,27 mbar 154 g z. T. ungesättigte Aldehyde (Substanz F; etwa 88 % d. Th.) über.

Die CO-Zahl des Produktes betrug 133 (theor. 131). Nach der gaschromatographischen Untersuchung wies das Produkt eine Reinheit von 97 % auf.

Beispiel 7

Hydroformylierung von Vinylnorbornen

In einem 5 l-Hubrührautoklaven wurden 180 g (1,5 Mol) Vinylnorbornen, 0,1 g (0,14 mMol) RhCl (CO) $[P(C_6H_5)_3]_2$ und 1,9 g (7,25 mMol) Triphenylphosphin miteinander vermischt. Der Autoklav wurde mit Synthesegas gespült. Anschließend wurde ein aus gleichen Volumina Wasserstoff und Kohlenmonoxid bestehendes Gemisch bis zu einem Druck von 200 bar aufgepreßt. Der Autoklaveninhalt wurde unter Rühren auf 90° C aufgeheizt, anschließend unter weiterem Rühren 5 Stunden lang auf 90 - 110° C gehalten und dann auf Raumtemperatur abgekühlt. Nach gaschromatographischer Analyse bestand das Rohprodukt aus 3 % nicht umgesetztem Olefin, 57 % Monoaldehyden und 40 % Dialdehyden.

Das nicht umgesetzte Olefin wurde im Wasserstrahlvakuum abgezogen. Bei der anschließenden Vakuumdestillation im Vakuum unter Stickstoffatmosphäre gingen nach dem Abtrennen der Monoaldehyde bei 78 - 95° C/0,27 mbar 60 g Dialdehyde (Produkt G; 25 % d. Th.) über.

Die CO-Zahl des Produktes betrug 287 (theor. 311). Nach der gaschromatographischen Untersuchung hatte das Produkt eine Reinheit von 96 %.

Beispiel 8

Hydroformylierung von Cycloocten

In einem 1 l-Hubrührautoklaven wurden 176 g (1,6 Mol) Cycloocten, 0,2 g RhCl (CO) $[P(C_6H_5)_3]_2$ und 3,8 g Triphenylphosphin miteinander vermischt. Der Autoklav wurde mit Synthesegas gespült. Danach wurde ein Gemisch aus gleichen Volumina Wasserstoff und Kohlenmonoxid bis zu einem Druck von 200 bar aufgepreßt. Die Reaktionsmischung wurde unter Rühren auf 110°C erhitzt, unter weiterem Rühren 4 Stunden lang auf dieser Temperatur gehalten und danach auf Raumtemperatur abgekühlt. Das erhaltene Rohprodukt bestand nach gaschromatographischer Analyse aus 4 % Olefin und Paraffin und 96 % Aldehyd. Bei der Vakuumdestillation unter Stickstoffatmosphäre gingen bei 93 - 94° C/21 mbar 194 g Aldehyd (Substanz H; 87 % d. Th.) über.

Die CO-Zahl des Produktes betrug 198 (theor. 200). Nach der gaschromatographischen Untersuchung wies das Produkt eine Reinheit von 99 % auf.

Beispiel 9

Antimikrobielle Wirkung der Substanzen A - H

Die antimikrobielle Wirkung der Substanzen A bis H bei der Flächendesinfektion wurde nach dem Scheuerdesinfektionstest ermittelt. Dieses Prüfverfahren ist den Richtlinien für die Prüfung chemischer Desinfektionsmittel, herausgegeben von der deutschen Gesellschaft für Hygiene und Mikrobiologie (1959), entnommen.

/14

Als Modelle für kontaminierte Flächen dienten 6 x 6 cm große Stücke von lackierten Holzplatten und PVC-Fußbodenplatten. Zur Kontamination der Flächen wurden eine Suspension von Candida albicans mit 2,7 x $10^8$ Keimen verwendet. Auf jede Testfläche wurde 0,1 ml Keimsuspension pipettiert und mit Hilfe eines Glasspatels gleichmäßig verteilt. Nach dem Antrocknen der Keimsuspension wurden die Testflächen mit Hilfe eines Wattetupfers gleichmäßig mit der Desinfektionslösung benetzt.

Zur Herstellung der Desinfektionslösungen dienten Konzentrate folgender Zusammensetzung:

10 Gew.-% Wirkstoff (Substanzen A bis H)
10 Gew.-% Nonylphenol + 9,5 Mol Ethylenoxid
40 Gew.-% Ethanol
40 Gew.-% Wasser.

Diese Konzentrate wurden zur Anwendung im Verhältnis 1 : 50 mit Wasser verdünnt.

Nach Einwirkungszeiten von 1, 2, 4 und 6 Stunden wurde jeweils ein Viertel der behandelten Flächen mit sterilen Wattetupfern abgerieben, die durch kurzes Eintauchen in Bouillon angefeuchtet waren. Die Tupfer wurden auf Bouillon-Agarplatten (Merck-Standard-I-Bouillon + 3 Gew.-% Tween 80 + 0,3 Gew.-% Lecithin + 0,1 Gew.-% Histidin) ausgestrichen. Die Agarplatten wurden 48 Stunden bei 30° C bebrütet.

Die bei der Auswertung gefundenen Ergebnisse sind in Tabelle I wiedergegeben.

Tabelle I

Abtötungszeit der Produkte A bis H für Candida albicans

| Produkt | Abtötungszeit in h | |
|---|---|---|
| | auf Holz | auf PVC |
| A | 6 | 6 |
| B | 4 | 6 |
| C | 6 | 6 |
| D | 6 | 6 |
| E | 6 | 4 |
| F | 2 | 4 |
| G | 6 | 6 |
| H | 6 | 6 |

/16

Beispiel 10

Nachfolgend werden einige Zubereitungen angegeben, in die die erfindungsgemäß zu verwendenden Aldehyde eingearbeitet wurden:

Antiseptisches Reinigungsmittel für Wäschereien

| | |
|---|---|
| Fettalkohol + 5 EO | 7 Gew.-Teile |
| Fettalkohol + 10 EO | 7 Gew.-Teile |
| Natriumtripolyphosphat | 32 Gew.-Teile |
| Natriumcarbonat | 9 Gew.-Teile |
| Natriumsulfat | 13 Gew.-Teile |
| Wasserglas | 5 Gew.-Teile |
| Natriumcarboxymethylcellulose | 1 Gew.-Teil |
| Substanz A | 7 Gew.-Teile |
| Wasser | 19 Gew.-Teile |

Desinfizierende Handwaschpaste

| | |
|---|---|
| Dodecyldimethylaminoxid | 10 Gew.-Teile |
| Kokosfettsäuremonoäthanolamid | 5 Gew.-Teile |
| Bimsstein, fein gemahlen | 60 Gew.-Teile |
| Tripolyphosphat | 5 Gew.-Teile |
| Substanz E | 0,5 Gew.-Teile |
| Glycerin | 2 Gew.-Teile |
| Lanolin | 2 Gew.-Teile |
| Carboxymethylcellulose, Na-Salz | 0,5 Gew.-Teile |
| Wasser | 15 Gew.-Teile |

Desinfizierendes Reinigungsmittel für harte Oberflächen

| | |
|---|---|
| $C_{10}$-$C_{12}$-Fettalkohol + 10 ÄO | 13 Gew.-Teile |
| Substanz H | 10 Gew.-Teile |
| Nitrilotriessigsäure, Na-Salz | 5 Gew.-Teile |
| Isopropanol | 10 Gew.-Teile |
| Wasser | 67 Gew.-Teile |

"Verwendung von Aldehyden als antimikrobielle Wirkstoffe"

Patentansprüche:

1. Verwendung von Aldehyden, die aus alicyclischen Kohlenwasserstoffen mit 6 bis 20 Kohlenstoffatomen und mindestens einer Doppelbindung durch Hydroformylierung in Gegenwart von Rhodiumkatalysatoren hergestellt werden, als antimikrobielle Wirkstoffe.

2. Verwendung von Aldehyden nach Anspruch 1, dadurch gekennzeichnet, daß die Aldehyde durch Hydroformylierung bei 70 bis 160° C unter einem Druck von 100 bis 400 bar und in Gegenwart eines Katalysatorgemisches aus tertiären Phosphinen und diese tertiären Phosphine enthaltenden Carbonylkomplexen hergestellt werden.

3. Verwendung von Aldehyden nach den Ansprüchen 1 und 2, dadurch gekennzeichnet, daß die Aldehyde durch Hydroformylierung von Limonen, Camphen, β-Pinen, α-Terpinen, Bicyclo-[2,2,1]-hepta-2,5-dien, dimeren Bicyclo-[2,2,1]-heptadien, Vinylnorbornen und Cycloocten hergestellt werden.

4. Verwendung von Aldehyden nach den Ansprüchen 1 bis 3, dadurch gekennzeichnet, daß man zur Herstellung von antimikrobiellen Mitteln die Aldehyde in Mengen von 0,1 bis 25 Gew.-%, vorzugsweise 0,5 bis 20 Gew.-%, bezogen auf das gesamte Mittel, einsetzt.

5. Verwendung von Aldehyden nach den Ansprüchen 1 bis 3, dadurch gekennzeichnet, daß man zur Konservierung die Aldehyde in Mengen von 0,1 bis 2 Gew.-%, bezogen auf das gesamte zu konservierende Produkt, einsetzt.

0018504

Europäisches Patentamt

# EUROPÄISCHER RECHERCHENBERICHT

Nummer der Anmeldung
EP 80 10 1702

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | US - A - 3 787 566 (C. GAUVREAU)<br>* Spalte 2, Zeilen 1-10; Spalte 3, Zeilen 57-75; Spalte 11, Anspruch 1 *<br>-- | 1-5 |
| | DE - A - 2 113 732 (HENKEL)<br>* Seite 38, Ansprüche 1 und 2 *<br>-- | 1-5 |
| | FR - A - 2 130 567 (MOBIL OIL)<br>* Seite 8, Anspruch 1 *<br>-- | 1-5 |
| A | FR - A - 2 371 192 (DRAGOCO)<br>-- | |
| A | FR - A - 2 310 135 (LES DERIVES RESINIQUES ET TERPENIQUES)<br>* Seite 2, Zeilen 12-14 *<br>-- | |
| E | EP - A - 0 011 272 (HENKEL)<br>* Ganzes Dokument *<br>-- | 1-3 |
| E | EP - A - 0 011 273 (HENKEL)<br>* Ganzes Dokument *<br>-- | 1 |
| E | EP - A - 0 008 459 (RUHRCHEMIE)<br>* Ganzes Dokument *<br>-- | 1 |

./..

**KLASSIFIKATION DER ANMELDUNG (Int.Cl. 3)**

A 01 N 35/02
C 11 D 3/48//
C 07 C 45/50
47/115
47/225
47/33
47/347
47/42
47/445
47/45

**RECHERCHIERTE SACHGEBIETE (Int. Cl. 3)**

A 01 N 35/02

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 25-08-1980 | PELTRE |

Europäisches Patentamt

# EUROPÄISCHER RECHERCHENBERICHT

Nummer der Anmeldung

EP 80 10 1702

-2-

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| A | FR - A - 2 224 428 (BASF) | 1-3 | |
| A | BE - A - 1 939 322 (JOHNSON, MATTHEY & CO) | 1-3 | |
| A | US - A - 3 965 192 (F.B. BOOTH) | 1-3 | |
| | ----- | | RECHERCHIERTE SACHGEBIETE (Int. Cl. 3) |